# EUROPEAN PATENT SPECIFICATION

(11) **EP 2 714 170 B1**
(45) Date of publication and mention of the grant of the patent: **16.03.2016**
(21) Application number: 12735951.1
(22) Date of filing: 25.05.2012
(51) Int. Cl.: A61M 16/04, A61M 25/09

(54) **Orotracheal tube for tracheostomy procedure**
Orotrachealtubus für Tracheostomieverfahren
Tube orotrachéal pour intervention de trachéotomie

(30) Priority: 25.05.2011 IT RM20110258
(43) Date of publication of application: 09.04.2014
(73) Proprietor: Servillo, Giuseppe, 80122 Napoli (NA) (IT); Tessitore, Gaetano, 81040 Francolise (CE) (IT)
(72) Inventor: Servillo, Giuseppe, 80122 Napoli (NA) (IT); Tessitore, Gaetano, 81040 Francolise (CE) (IT)
(74) Representative: Modiano, Micaela Nadia
(86) International application number: PCT/IT2012/000154
(87) International publication number: WO 2012/160585

(56) References cited:
- GB-A- 708 477
- US-A- 4 769 005
- US-A- 5 507 279
- US-A- 5 807 330
- US-A1- 2006 032 505
- US-A1- 2006 081 260
- US-A1- 2007 017 527
- US-A1- 2007 163 596
- US-B1- 6 443 156

## Description

The present invention relates to an orotracheal tube for interventions of tracheotomy.

As it is well known, the control of airways is a characteristic of the treatments administered to patients under intensive care. In many cases, when the functionality of the respiratory system is compromised for several reasons, only an invasive control of the patient airways ensures adequate gas exchange. This airway management under intensive care can be achieved through the translaryngeal nasal/oro/tracheal intubation or by tracheotomy. The performance of tracheotomy in patients under intensive care is of secondary type, i.e. elective and not-urgent, so the risk/benefit ratio and the most appropriate timing are set on a case by case basis. With regard to indications, there are four main reasons why the medial operator to choose a tracheostomy: the need for a long-term mechanical ventilation, the failure of weaning from mechanical ventilation, upper airway obstruction and the presence of abundant secretions. These signs include a vast amount of pulmonary and non pulmonary diseases, which is frequently found in patients in intensive care. In particular, the guidelines for the management of the airways of patients undergoing mechanical ventilation implement the tracheostomy in intensive care saying that after the first seven days of mechanical ventilation it is necessary to assess the possibility to extubate the patient in the following seven - ten days. If the answer is positive it is possible to continue in the conventional way, but if the answer is negative it is advisable to plan the tracheotomy.

Moreover, if it is foreseen that the residence time of artificial airway exceeds 21 days, proceeding to tracheotomy as soon as possible is a must. When it is not possible to predict the timing of artificial respiration, the patient should be evaluated daily to determine when the tracheotomy becomes indicated.

Absolute contraindications to this method are skin infections and previous major surgery that alters the neck and completely obscures the anatomical relationships.

The tracheotomy in intensive care offers many advantages, improves comfort and reduces the demand for sedation of the patient. In an observational study, tracheotomized and mechanically ventilated patients in intensive care needed a smaller dose of sedatives, spent less time sedated and reached full autonomy earlier [6]. Furthermore, a tracheotomy can offer patients the opportunity to articulate speech, which may reduce anxiety and increase well-being. Another advantage of this technique is the opportunity for oral feeding, in fact, although swallowing and barrier function performed by the epiglottis may be compromised, with the necessary precautions oral nutrition is not prevented [8]. The tracheotomy may also facilitate weaning from mechanical ventilation, reducing the dead space, the airway resistance, work of breathing (also called WOB) and improving the removal of secretions. Anatomical dead space includes that portion of inhaled air that does not reach the alveoli, but remains in the conducting airways, not participating in gas exchange. In adults it is quantified in about 150ml or 30% of inspired volume. This quantity is definitely greater than the volume that remains inside a tracheotomy cannula which is approximately 5ml [6]. Furthermore, the difference between the dead space offered by the endotracheal tube and a tracheotomy cannula of the same size is less than 20ml [9]. Therefore, it is unlikely that this small change in dead space has a significant role in facilitating weaning from mechanical ventilation, however, it is helpful in case of respiratory precarious equilibrium. In contrast, a reduction of the work of breathing due to a decrease of the resistance offered to the inspiratory flow, could facilitate the weaning process. It has also been shown that the work of breathing is less with a tracheotomy cannula with respect to an endotracheal tube of the same diameter [9]. This difference increases with increasing inspiratory flow. This finding was further confirmed *in vivo* by the same authors who have also found a reduction of pressure at the end of the respiratory cycle (positive end-expiratory pressure or PEEP), which is intrinsic after tracheotomy [9]. An even more marked reduction of intrinsic PEEP and WOB were recorded, both in spontaneous breathing and in breath-assisted (pressure support) breathing, even in patients with chronic obstructive pulmonary disease (COPD) who had previously failed the weaning process [10].

Recently, the latest recommendations regarding tracheotomies suggest that patients destined to a long-term mechanical ventilation should be tracheotomized early (within the first week) in order to reduce the duration of artificial ventilation itself and recovery in intensive care unit (grade 1 B) [6].

According to traditional techniques, the tracheotomy itself was a real surgery intervention, leading to skin incision, which included skin, subcutaneous cellular tissue and superficial muscles. Modern techniques of percutaneous tracheotomy have been introduced in the '80s as alternative to the surgical technique [3]. In 1985, Ciaglia introduced the technique of percutaneous dilatational tracheotomy (or percutaneous dilated tracheotomy PDT), which provided for a progressive dilation of the tracheostoma using blunt-tipped dilators [4].

The percutaneous tracheostomy technique introduced by Ciaglia in 1985 has notably simplified the technique, moving the execution of the same from the operating room to the bedside, even and especially in intensive care units. The technique of percutaneous dilated tracheotomy offers several advantages over the prior purely surgical (surgical tracheotomy or ST). Its execution in intensive care eliminates the risk of transport of critically ill patients and the costs of use of operating room [11]. Compared to surgical technique, the technique of percutaneous dilatational tracheotomy may be associated with fewer peri-and post-surgery complications, including bleeding and infection, as has been suggested by a recent meta-analysis [12]. Because of these potential advantages, the popularity of the technique of percutaneous dilatational tracheostomy has increased significantly in recent years [13].

In particular, the technique devised by Ciaglia in 1985 is based on the idea of adapting to the trachea multiple dilators derived from percutaneous nephrostomy kit [14]. In the original technique the cricoid cartilage was identified and from the bottom edge thereof the skin was cut vertically for 1 to 1.5 cm [14]. More recent studies have shown that this subcricoidal access, being too high, increased the risk of subglottic stenosis [4]. Currently it is preferred to cut between the first and the second or between the second and the third tracheal ring [4]. After the skin incision, a cannula-needle connected to a syringe containing normal saline solution is inserted into the trachea at the level of the median line. The correct insertion of the cannula is confirmed by drawing air into the syringe containing saline solution. After removal of the needle, through the cannula is inserted a guide wire on which first passes a small dilator and then a second guide being a teflon 8French (2.64 mm) graduated guide. This guide makes the system more stable of the guide wire, preventing its flexion and the risk of creating a false paratracheal road. On both 7 serial blunt-tipped dilators of increasing diameter (12-36French, 3.96 to 11.88 mm) are slided to obtain a suitable means for the passage of the cannula.

During all steps of the procedure endoscopic control can be used.

The technique of Ciaglia can be chosen for those patients with an anatomy of the cervical region that allows easy identification of reference points, with no alterations of coagulation and, above all, without predictive criteria of difficult intubation [11]. The technique of percutaneous dilatational tracheostomy does not seem appropriate for those patients with abnormal anatomy of the neck, maxillofacial trauma or refractory coagulopathy [11]. Also the gradual expansion is not made without difficulty. The technique requires a considerable pressure and stress on the tissues of the neck, which may lead to damage to the rear wall of the trachea. Such damage can also be attributed to inadequate stabilization of the Teflon guide and the guide wire (Seldinger type) during insertion of the tube [15].

In addition, during insertion of the dilator and the cannula, the tracheal lumen temporarily collapses and compresses about 90% of the airway [15]. According to a recent report studying 500 cases of tracheotomy, the most frequent complications that occur during the technique of Ciaglia are the desaturation and bleeding [16].

The technique of Ciaglia was subsequently improved by the same Ciaglia inn 1999, providing for the use of a single tapered dilator (called blue-rhino by virtue of its curved horn shape) with the aim of obtaining dilation of the tracheal wall with a single progressive movement. The curved horn with soft tip is advanced on the teflon guide up to bring a black mark of reference of which it is provided in correspondence with the skin (the black mark indicates the point at which the dilator reaches a diameter of 12.6 mm): The dilator is covered with a hydrophilic coating that facilitates slippage between the tissues.

The blue rhino technique, according to the author, should be faster and safer than the original technique. The procedure may also be completed in five minutes if there were problems with the tracheal puncture of with display via bronchoscope [17]. According to further studies, however, this procedure is not without bleeding, but that would occur in 14% of cases [18].

Another technique was developed by Griggs in the early '90s, and proposes the use of a Howard-Kelly forceps, modified with curved prongs, blunt and grooved so as to proceed on a guide wire [19]. The tracheal incision extends for about 1.5-2cm below the lower border of the cricoid cartilage. A 14gauge (1.628 mm) cannula-needle, connected to a syringe containing normal saline solution, is advanced through the incision until the presence of air bubbles in the syringe does not confirm the positioning in the trachea. After removing the needle, a Seldinger type guide wire with J-tip is advanced into the trachea through the cannula. After removal of the cannula, a first dilator slides on the guide wire dilating the cervical tissues and the anterior wall of the trachea. Subsequently, the first dilator is replaced with the gripper that opens first in the pre-tracheal tissues and then in the front wall to make it easy the introduction of the tracheal cannula [20]. This technique can be performed under endoscopic guidance.

The technique of Griggs can be carried out quickly at the patient's bed. The use of a bronchoscope may make it more secure and reduce the complications [20]. In fact, direct vision of the interior of the trachea, possible with the endoscopic control, allows to avoid damage to the rear wall keeping under control the movement of the different instruments used [20]. However, the use of the bronchoscope itself can lead to retention of CO₂ intra-operatively, so it may be contraindicated in those patients who can not tolerate this increase [21]. The most common short-term complications of this technique are, again, bleeding and infection of the stoma [20].

A different and unique technique was introduced by Fantoni and provides that the expansion and the positioning of the cannula is to take place from the inside to the outside of the trachea (translaryngeal tracheostomy technique). Initially, after removal of the endotracheal tube, a rigid rectilinear tracheoscope is inserted into the trachea. Within this is inserted an optical fiber at 0°, which allows visualization of the chosen interannular space and of the vocal cords by transillumination. The selected area is then push towards the outside to make the tracheal axis more linear, make the layer of the tissues of the neck thinner and more easily identify from the outside the tip of the tracheoscope. The rigidity of the tracheoscope prevents lateral and downwards movements in the trachea, allowing to introduce a curved cannula-needle in its interior. The introduction of this rieedle (in a point more distal from the selected one), even if curved, however does not protect from the risk of damaging the rear wall. A guide wire, which terminates with a J-type segment (Seldinger type guide wire, in the following, more simply called, according to the terminology of the industry: Seldinger), is inserted through the needle into the tracheoscope (maneuver difficult to perform) and is continued until exiting from a fitting that connects the tracheoscope with the ventilation system, where it is grasped by the operator.

At this point, the needle is slipped off, holding the end of the guide wire with a gripper outside the oral cavity of the patient. After removal of the needle, it is also extracted the tracheoscope. At this time, the patient is not ventilated and there is no possibility to ventilate until a new intubation is not carried out, which is realized with a small endotracheal tube (inner diameter 5 mm) uncuffed in the lower third of the trachea, which allows ventilation of the patient. According to this method, it is therefore expected a double intubation: the first with a tracheoscope drive, the second with an endotracheal tube. At this point, while continuing to ventilate the patient, the end of the guide wire retained outside the oral cavity is inserted into a tracheal cannula provided with a conical end portion with a metal tip with an axial through hole, which allows the insertion inside of the Seldinger guide of the guide wire, which is made to exit from the opposite end of the cannula. At the end of the guide wire is practiced a node, which will form an enlargement such as to prevent the guide wire itself may fall out of the metal tip, making a backward path compared to that of its insertion into the cannula, while its distal end (which protrudes from the tracheal wall) is rolled up on a knob extraction. At this point, the traction of the distal end of the guide wire start, in order to slide the cannula within the oral cavity, through the vocal cords, up to reach the anterior tracheal wall. At this level, the operator, from the outside, affix two fingers around the guide wire and exerts a back pressure at the exit end of the cannula towards the outside. The extraction of the cannula must continue to a point such as to allow its rotation inside the trachea, position shown by the emrging of appropriate reference lines of which said cannula is provided. Having done this, the conical portion of the cannula is cut, to the end of which the metal tip is present, and a shutter is introduced in it which makes the cannula linear and allows to turn its axis by 180°, an operation facilitated by the presence of a line of reference which, at the end of operation, will be facing upwards. Finally, the cannula is pushed back into the trachea, returning to follow its natural curvature [22].

The tehnique of Fantoni is not devoid of critical points. A first critical point is the inclusion of tracheoscope, which can be difficult in cases of difficult intubation [22]. Other sensitive issues are the insertion of the curved needle, the insertion of the guidewire into the lumen of the tracheoscope, the extraction, straightening and turning of tracheal cannula, maneuvers that are often difficult and may lead to the extraction of complete prosthesis itself (and thus the risk of giving up or to recommence the procedure) [23]. The same straightening of the trachea, the repeated punctures of the introducer needle (creating a weak point) and excessive pressure on this point at the time of the passage of the cannula may lead to breakage of the selected tracheal annulus. The ventilation with the endotracheal tube is associated with an increase in pressure of CO₂ that has been encountered at the end of the method, probably due to a difficulty of the exhaust air (which requires special attention with some categories of patients) and forces the operator to an increased ventilation (jet ventilation) that could cause pneumothorax in selected patients [22, 23]. Furthermore, this technology involves increasing the pressure at the end of the intrinsic respiratory cycle pressure (PEEP), so that, if the procedure is not performed with sufficient speed, the condition of the patient's ventilatory become critical. However, the tight fit of the tracheal cannula to reduce the risk of bleeding, infection and dehiscence of the stoma, found in other percutaneous and surgical methods [5], which makes the technique invented by Fantoni the translaryngeal tracheotomy technique with less complications at a distance.

In conclusion, a problem common to all the various techniques of tracheotomy is that they perform all downstream of the orotracheal tube, some (Fantoni) at least in the initial part, other (surgical) in the final part, other throughout the procedure (dilated)

In the light of the above, it appears evident the need to have available an apparatus that allows the execution in safety of a tracheotomy, regardless of the technique used, eliminating the problems related to the methods of the prior art.

US2007/017527 discloses a device and method for providing a disposable endotracheal intubation device for use with an auxiliary passageway serving as a guide for the placement of an orogastic or other enterally directed device in a patient.
US2006/032505 discloses a Perilaryngeal oral airway with multi-lumen etophogeal-obturator.
US6443156 B discloses a separable double lumen endotracheal tube.

In this context it is proposed the solution according to the present invention, which aims to provide an oro-tracheal tube with at least ventilatory and preferably polyfunctional operation, which achieves both the ventilatory function and the operational phases, which can be inserted easily and at the same time ensures an optimal ventilation of the patient throughout the procedure and allows to define the surgical field between the larynx and the trachea near the bottom, separating the surgical field from the lower respiratory tract, thus avoiding that the same may be blocked by bleeding or secretions and at the same time providing a protective wall of the rear wall of the trachea (safeguarding the patient's life at every moment of the procedure), since it occupies only the rear part of the trachea.

According to preferred embodiments, the oro-tracheal tube of the present invention can carry out simultaneously the ventilatory and operational function, allowing the insertion of the optical fibers operating in a lumen that does not interfere with the ventilatory, and allowing to follow the entire tracheotomy procedure in direct vision, in accordance with any methodology of tracheotomy, avoiding the double intubation necessary in the technique of translaryngeal tracheotomy conceived by Fantoni (and in this way, safeguarding the patient's life because there is no time during the procedure in which the patient is not ventilated or even can not be ventilated), protecting the rear wall of the trachea, allowing to operate quietly and follow the entire procedure in direct vision through the use of a bronchoscope.

These and other results are obtained according to the present invention suggesting a oro-tracheal tube which has a length such as to arrive in the vicinity of the hull and that, at least in the distal tract, ie in the portion inserted deeper into the trachea, has a flattened section, or smaller along the sagittal axis, and more extended along the transverse axis and which, in the vicinity of the distal portion is provided with a headset which is asymmetrical with respect to the tube, which pushes the tube to approach towards the rear of the trachea.

Preferably, to allow the use in tracheotomy avoiding the need for a double intubation, which is instead necessary in the technique of translaryngeal tracheostomy by Fantoni, as well as ventilation problems due to a precarious dislocation of the tube and the interference of the optical fibers that occur in the technique of percutaneous tracheostomy conceived by Ciaglia, said oro-tracheal tube is equipped with two or three lumens, in the case of two lumens respectively for performing the ventilatory and operational functions, in the case of three tubes to provide an additional passage dedicated for instance to a system of aspiration of secretions and blood from the surgical field.

Moreover, in a further form of the present invention, the oro-tracheal tube according to the present invention is associated with a modified tracheal cannula, even in this case to allow in particular to overcome the problems associated with the extraction, the straightening and the turning of the tracheal cannula during application of the technique of translaryngeal tracheostomy conceived by Fantoni.

The purpose of the present invention is therefore to provide a orotracheal tube that allows to overcome the limits of the solutions according to the prior art and to obtain the technical results previously described.

Further object of the invention is that said oro-tracheal tube can be manufactured with substantiatly low costs, both as far as production costs is concerned and as far as the maintenance costs is concerned.

Another object of the invention is to provide an oro-tracheal tube which is substantially simple, safe and reliable.

It is therefore a first object of the present invention an oro-tracheal tube as defined in claim 1.

Further characteristics of the oro-tracheal tube of the present invention are defined in the subsequent dependent claims.

Also forms a second specific object of the present invention a kit that includes the oro-tracheal tube for tracheotomy according to the present invention, as defined in claim 6.

It is evident the effectiveness of the oro-tracheal tube of the present invention, which allows to pursue the results in charge and in particular is an advantage compared to the techniques of tracheotomy of the prior art, surgical, percutaneous or translaringel, with reference to the ease of execution, safety for the patient (thanks to the delimitation of the operating field and the separation of the same from the lower respiratory tract, but also to the direct visual control in all phases of the intervention and possible aspiration of secretions and blood from the surgical field) and to the reduction costs.

Where in the following the word invention is used and/or features are presented as optional this should be interpreted in such a way that protection is sought for the invention as claimed.

The present invention will now be described, for illustrative but not limitative purposes, according to its preferred embodiments, with particular reference to the figures of the accompanying drawings, in which:
- Figure 1 shows a side view of an oro-tracheal tube,
- Figure 1A shows a cross-sectional view of the oro-tracheal tube of Figure 1, in correspondence of the section line A-A',
- Figure 1 B shows a cross-sectional view of the oro-tracheal tube of Figure 1, in correspondence of the section line B-B',
- Figure 1C shows a cross-sectional view of the oro-tracheal tube of Figure 1, in correspondence of the section line C-C',
- Figure 1 D shows a cross-sectional view of the oro-tracheal tube of Figure 1, in correspondence of the section line D-D',
- Figure 1 E shows a cross-sectional view of the oro-tracheal tube of Figure 1, in correspondence of the section line E-E',
- Figure 1 F shows a cross-sectional view of the oro-tracheal tube of Figure 1, in correspondence of the section line F-F',
- Figure 2 shows a side view of an oro-tracheal tube,
- Figure 2A shows a cross-sectional view of the oro-tracheal tube of Figure 2, in correspondence of the section line A-A',
- Figure 2B shows a cross-sectional view of the oro-tracheal tube of Figure 2, in correspondence of the action line B-B',
- Figure 2C shows a cross-sectional view of the oro-tracheal tube of Figure 2, in correspondence of the section line C-C',
- Figure 2D shows a cross-sectional view of the oro-tracheal tube of Figure 2, in correspondence of the section line D-D',
- Figure 2E shows a cross-sectional view of the oro-tracheal tube of Figure 2, in correspondence of the section line E-E',
- Figure 2F shows a cross-sectional view of the oro-tracheal tube of Figure 2, in correspondence of the section line F.-F',
- Figure 3 shows a side view of an oro-tracheal tube according to first embodiment of the present invention,
- Figure 3A shows a cross-sectional view of the oro-tracheal tube of Figure 3, in correspondence of the section line A-A',
- Figure 3B shows a cross-sectional view of the oro-tracheal tube of Figure 3, in correspondence of the section line B-B';
- Figure 3C shows a cross-sectional view of the oro-tracheal tube of Figure 3, in correspondence of the section line C-C',
- Figure 3D shows a cross-sectional view of the oro-tracheal tube of Figure 3, in correspondence of the section line D-D',
- Figure 3E shows a cross-sectional view of the oro-tracheal tube of Figure 3, in correspondence of the section line E-E',
- Figure 3F shows a cross-sectional view of the oro-tracheal tube of Figure 3, in correspondence of the section line F-F',
- Figure 4 shows a side view of an oro-tracheal tube according to a second embodiment of the present invention,
- Figure 4A shows a cross-sectional view of the oro-tracheal tube of Figure 4, in correspondence of the section line A-A',
- Figure 4B shows a cross-sectional view of the oro-tracheal tube of Figure 4, in correspondence of the section line B-B',
- Figure 4C shows a cross-sectional view of the oro-tracheal tube of Figure 4, in correspondence of the section line C-C',
- Figure 4D shows a cross-sectional view of the oro-tracheal tube of Figure 4, in correspondence of the section line D-D',
- Figure 4E shows a cross-sectional view of the oro-tracheal tube of Figure 4, in correspondence of the section line E-E',
- Figure 4F shows a cross-sectional view of the oro-tracheal tube of Figure 4, in correspondence of the section line F-F',
- Figure 5 shows side view of a tracheal cannula of a translaryngeal tracheostomy kit for the modified Fantoni, method,
- Figure 6 shows a bottom view of the element of introduction of the removable tracheal cannula of Figure 5,
- Figure 7 shows a top view of the removable introduction of the tracheal cannula of Figure 5,
- Figure 8 shows an enlarged view of the tip of the removable introduction element of the tracheal cannula of Figure 5,
- Figure 9 shows a top view of a shutter of a translaryngeal tracheostomy kit for the modified Fantoni method,
- Figure 10 shows a side view of the shutter of Figure 9,
- Figure 11 shows a side view of a Seldinger guide wire with a metal wire of a translaryngeal tracheostomy kit for the modified Fantoni method
- Figure 11A shows an enlarged side view of the detail A of the Seldinger guide wire and the metal wire of Figure 11, and
- Figure 12 shows a side view of a screw-cap at the end of the wire of Figure 11.

The fundamental element of the proposed modification of the technique of tracheotomy is certainly the oro-tracheal tube designed according to the present invention.

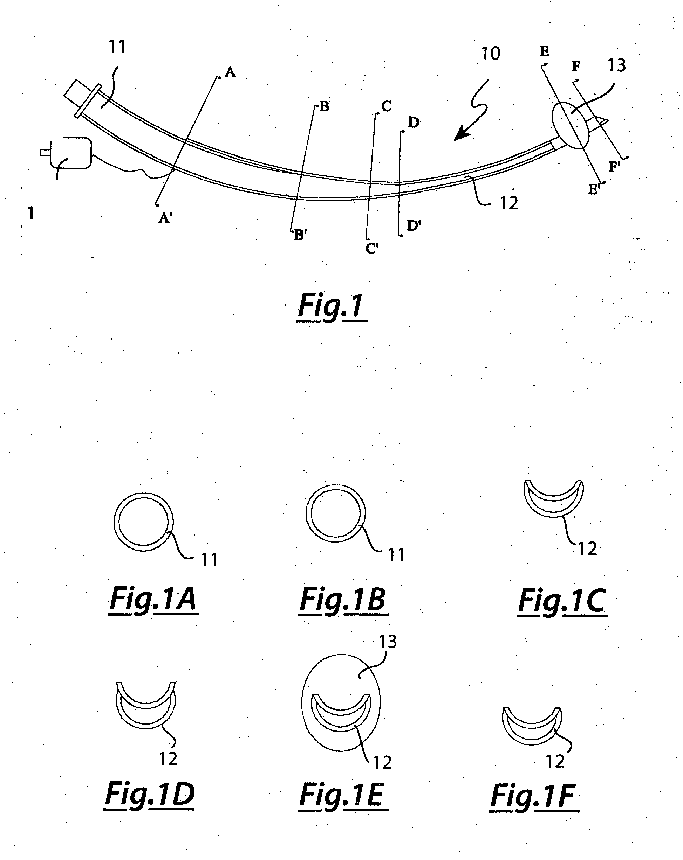

Referring to Figures 1, 1A-1F, the oro-tracheal tube is designated as a whole by the reference numeral 10 and is constituted by a single body, made, by illustrative but not limitative way, with plastomeric material, or PVC , or silicone, or a material with memory shape, and which may be optionally reinforced with steel wire to allow maximum flexibility of the tube in the longitudinal direction, at the same time giving rigidity to the shape of its cross section. In addition, the material can also be equipped with screens which allow the use of an electrosurgical unit.

Depending on the rigidity of the material with which it is made, to facilitate insertion into the trachea, the oro-tracheal tube according to the present invention may be preformed with a physiological curvature in the longitudinal direction.

The section of the oro-tracheal tube 10 shown with reference to Figures 1, 1A-1F is of variable size and shape as proceeding in the longitudinal direction along the tube: circular in the proximal portion 11 of the orotracheal tube 10, ie in the part that protrudes from the mouth of the patient, and crescent in the distal portion 12, ie in the part that is inserted deeper into the trachea. Finally, the orotracheal tube 10 is provided, proximate to its distal end, with an inflatable cuff 13, connected to an inflation system 14 comprising a tank with one-way valve, through which the air is injected to inflate the cuff 13. Alternatively, the cover 13 may be lacking of the inflation system, but rather consists of self-expandable material, or shape memory or other equivalent material.

The inflatable cuff 13 is asymmetric, in particular inflating more in an anterior direction, thus pushing the oro-tracheal tube 10, or at least its distal portion 12, on the rear wall of the trachea. The pushing action exerted by the cuff 13 on the distal portion 12 of the oro-tracheal tube 10, together with the flat shape of the section of the same distal portion 12, allow to leave the trachea as free as possible" or to leave a space sufficient to perform the tracheotomy, according to any one of the surgical, percutaneous or translaringeal methods.

In particular, the oro-tracheal tube 10 has sufficient length to arrive down to the vicinity of the hull, where, after insertion, is fixed through the inflation cuff 13 by means of the inflation system 14.

The distal portion 12 of the oro-tracheal tube 10, with a crescent shape, serves primarily to assist the ventilation of the patient throughout the procedure, but its crescent shape and its length also have an additional function, the oro-tracheal tube being inserted in the trachea so that the concave outer surface of the distal portion 12 is facing the front wall of the trachea, in this way constituting a real passage seat which protects the rear wall of the trachea, preventing other devices used in the course of tracheotomy could accidentally hit the back wall of the trachea, and providing at the same time, thanks to its crescent shape, an obliged lane to follow.

In particular, in addition to the shape in section, also the gauge of the oro-tracheal tube varies in the longitudinal direction.

Still more in particular, the initial portion, ie the one that starts from the outside of the mouth and reaches the larynx, in the embodiment shown in Figure 1, has a normal cylindrical shape, but could also have other forms, which allow in any case a good outflow of the air, with a measure of internal diameter Variable but such as to allow the insertion into the trachea.

The distal portion 12, which continues from the larynx to the vicinity of the hull (of length ranging from 50mm up to 85mm depending on the length of the neck of the patient) can be realized with a cross section of different forms, all, however, crushed, or more extended in transverse direction rather than in sagittal direction. In particular, the shape of the section of the distal portion 12 may be, by way of example but not limitated to, crescentic or elliptical or even rectangular with rounded corners, and in any case can take all those forms that may encumber the least possible the trachea, leaning on the rear wall of the same and at the same time protecting it, and which allow at the same time a good ventilation of the patient (even in mechanical ventilation), with the only limitation that the major axis of the section does not exceed the inner diameter of the trachea.

In the oro-tracheal tube 10 shown in Figures 1, 1A-1F, the proximal portion 11 and the distal portion 12 are connected by a connecting portion long enough to allow a slow variation of the section, for not interfering with the air (or mixture of air enriched with oxygen or other elements chosen according to the type of operation and the needs of the patient) that passes through the tube, in particular avoiding to set up a resistance to the passage.

As to the distal portion 12, of flattened shape, to obtain a good ventilation which makes the pipe suitable for each type of ventilation, it will preferably have a ventilation area equal to or higher than a classic cylindrical orotracheal tube with an inside diameter equal to 6,5 mm, ventilation area that allows a normal ventilation, both manual and mechanical, in all types of patients.

In conclusion, the oro-tracheal tube according to this embodiment enables to obtain the following results:
- good ventilation of the patient, with excellent saturation of oxygen;
- good elimination of CO₂, which remains within the normal range;
- no increase in intrapulmonary pressure;
- no increase in intracranial pressure;
- no periods of apnea d'uring the intervention, and
- absolute protection of the rear wall of the trachea.

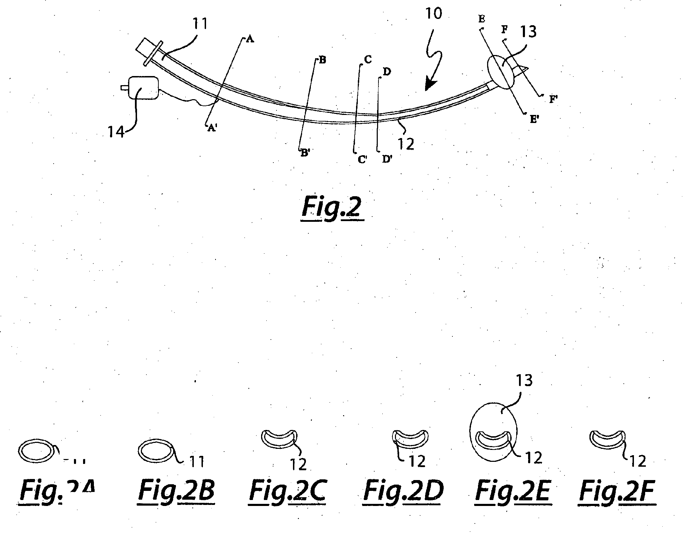

Figures 2, 2A-2F show the orotracheal tube which, compared to that shown in Figures 1, 1A-1F differs due to the fact that even the proximal portion 11 has flattened shape.

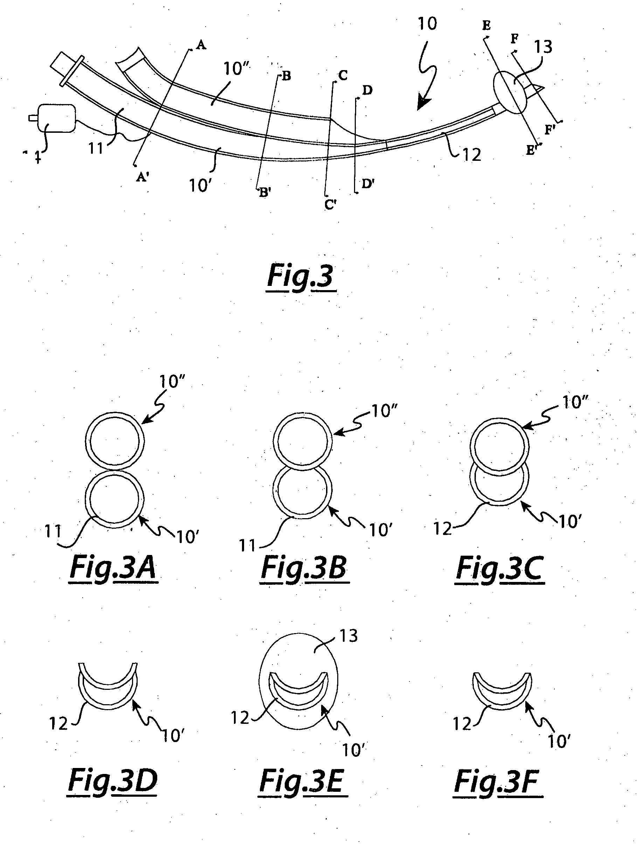

In a first embodiment, shown with reference to Figures 3, 3A-3F, the oro-tracheal tube 10 according to the present invention has two lumens, respectively, determined by a rear tubular element 10' (in the rest of the description also referred to as lower tubular element 10' or ventilatory tubular element 10'), intended to allow for ventilation of the patient during all phases of operation and to separate the operative field from the lower airways and realized according to what has already been previously described with reference to Figures 1, 1A-1F, ie with a flattened shape at least in the distal portion 12 and equipped at its end with an inflatable cuff 13, connected to an inflation system 14 comprising a tank with one-way valve, through which the air is injected to inflate the cuff; as well as by a front tubular element 10" (in the rest of the description also referred to as upper tubular element 10" or operative tubular element 10") of different shape and size depending on the technique of tracheotomy for which the oro-tracheal tube according to the present invention is made.

In particular, the front tubular element 10" can have circular cross-section (if intended to be used for translaringeal tracheotomies), with an inner diameter preferably equal to 13 mm, to allow the passage to the inside of a tracheotomy tube of the type already used in accordance the prior art and previously described in this patent application, or it can have an oval section (if intended to be used for dilatation type tracheotomies), with transverse axis preferably equal to 10 mm and sagittal axis preferably equal to 8mm.

In all variations, however, the front tubular element 10" is shorter than the rear tubular element 10', so that when its proximal end remains just outside the mouth, its distal end reaches amongst the vocal cords, or, in a variant thereof, exceeds them slightly.

By examplificative but not limitative way, with reference to the technique of translaryngeal tracheostomy conceived by Fantoni, the function of this lumen is to allow in succession the following operations: the insertion of means of transillumination, the insertion of the guide wire, the subsequent insertion of the tracheal cannula and the insertion of the same tracheal cannula, and the insertion of the bronchoscope in order to follow the entire procedure under direct vision.

Also according to this embodiment, the rear tubular element 10', of flattened shape at least in the distal portion 12; defines a lumen connected to a ventilation system and serves primarily to assist the ventilation of the patient throughout the procedure. The flattened shape of the rear tubular element 10', disposing around the distal portion of the fron tubular element 10", allows the realization of a lumen that responds in a perfect manner at the same time to two needs, have an optimum ventilatory surface and at the same time occupy the as little space as possible in the trachea, creating the ideal compromise for playing then both the ventilatory and the operational function, and at the same time allow the execution of the method up to the final positioning of the tracheotomy tube by holding the tube fixed into the trachea; compromise impossible to obtain with the arrangement of two cylindrical tubular elements placed side by side according to the prior art.

In addition, the flat and preferably crescentic shape rear tubular element 10', its position with respect to that of the front tubular element 10"and its greater length also have an additional function, the outer surface of the rear tubular element 10' constituting a real passage seat which, by way of example without limitation of the present invention, by referring to the technique of translaryngeal tracheotomy conceived by Fantoni, facilitates, after insertion of the guide wire, its channeling into the lumen defined by the front tubular element 10", coming out easily at the proximal end, outside the mouth, avoiding that the guide wire itself can accidentally hit the back wall of the trachea, protecting it, and providing the wire itself, thanks to its crescentic shape, one obliged lane to follow.

Furthermore, said shape of the tubular element 10', for the dilatative percutaneous technique (Ciaglia), avoids the lesions of the rear wall; which is possible with the cannula, the pre-dilator, the dilator etc.., representing a guide on which the Seldinger guide slides toward the bottom (hull); allows excellent ventilation, protects the airways.

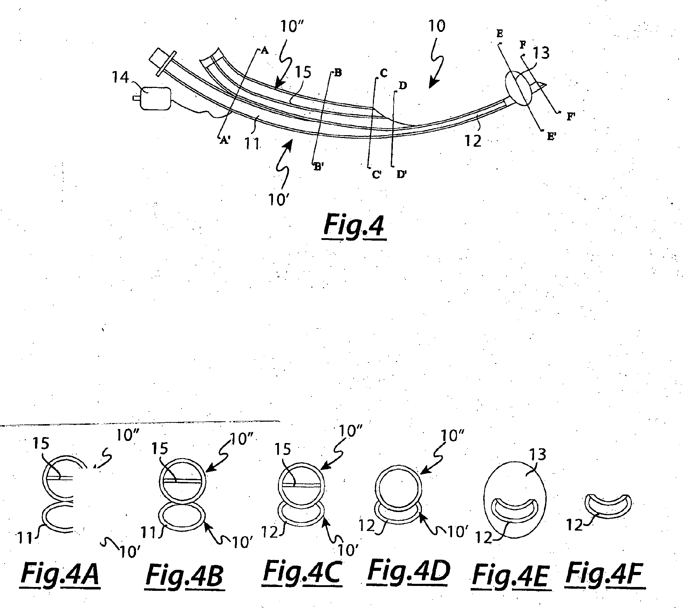

Referring to Figures 4, 4A-4F, the oro-tracheal tube according to the present invention is shown according to a further embodiment which differs from that shown in Figures 3, 3A-3F for the fact that the front tubular element 10'' is divided into two lumens by a septum 15 transverse to the sagittal plane (but can also be sagittal) and as long as the front tubular element 10". In this case, therefore, the oro-tracheal tube according to the present invention is, in complex, a tube with three lumens.

The division into two lumens of the front tubular element 10" allows the simultaneous insertion, into two mutually independent lumens, of a fiber optic system, which allows to perform the surgery under direct vision, and a suction tube, which allows to keep the operative field clean.

The advantages offered by the oro-tracheal tube according to the present invention, in the embodiment that provides for the presence of a rear tubular element 10', and more generally of a lumen dedicated solely to the ventilation of the patient, and a front tubular element 10", and more generally of one or two lumens dedicated solely to operational requirements and which do not interfere in any way with the ventilation of the patient, are different:
- the patient is intubated only once and not twice (as is necessary according to the method of Fantoni);
- intubation, being preferably obtained not through a rigid tube (as according to the method of Fantoni), but through a flexible tube, possibly with a preformed physiological curvature, is certainly simpler, easier to perform (especially in difficult intubation), and no less important, less traumatic to the patient;
- inside the tube, through the operative lumen defined by the front tubular element 10", it is possible to use a flexible fiber optic system, which is also less traumatic than rigid optics according to the prior art;
- the optical fibers come out below the vocal cords, between the first and the second tracheal ring, where the stoma is usually practiced;
- it is easier to carry out the transillumination, always between the first and second tracheal ring, and more generally where one has to practice the puncture of the trachea;
- it is also simpler the execution of the puncture of the trachea (major limitation of the method of Ciaglia), in that under the transillumination, the upper wall of the trachea is free from the tube, but at the same time the rear wall of the trachea is protected by the rear tubular element;
- the optical fibers, inserted in the operative lumen, allow to follow in direct vision (without interfering with the ventilatory lumen, as is the case for the method of Ciaglia) the entry into the trachea of the cannula-needle the Seldinger, the dilator, and finally of the tracheal cannula used in the manner already described with reference to the prior art;
- it is possible to insert, also at the same time, even in the same lumen, or in a separate lumen in the case of an oro-tracheal tube with three lumens, a suction system, which always keeps the operating area free from secretions and blood, allowing always good vision.

In particular, with reference to the technique of tracheotomy by Fantoni, the oro-tracheal tube of the present invention allows to achieve further specific advantages:
- through the operative lumen defined by the front tubular element 10", the introduction of optics is easier, and it is easier to carry out the subglottic transillumination, where it is inserted, through a cannula-needle, the guide wire;
- the guide wire easily is directed cranially also guided by the rear tubular element 10', which, in addition to protecting the rear wall of the trachea, acts as a real guide;
- once the guide wire leaves from the end of the lumen defined by the front tubular element 10", the cannula 20 is joined, as explained in detail in the following description, and without removing the oro-tracheal tube 10, it slips the cannula through the lumen;
- while continuing to pull the cannula 20 making it out from the trachea with the tip 21, in the oro-tracheal tube 10 a flexible optic can be introduced that, from this moment on, will allow to follow the subsequent steps in direct view; this is impossible with the apparatus according to the prior art.

Moreover, the possibility of insertion of flexible optics introduces the great advantage of visually following the sliding, the revolution, and the exact positioning of the cannula 20 (not possible with the prior art), it is inserted and retained in the trachea until the correct positioning of the tracheotomy tube, because of its particular shape leaves enough space to perform all the operations of introduction and turning. Immediately after the insertion of the cannula 20 and its proper positioning, after deflation of the cuff 14, the tube 10 is withdrawn leaving the cannula in place. In this way it is possible to avoid the risk of the cannula to be fully ejected from the trachea, compromising the entire operation, as instead can happen according to the prior art, since it does not allow to visually follow the operation.

Finally, other advantages are associated with the crescentic shape of the rear tubular element 10", that, even with respect to the thin and long ventilation tube included in the Fantoni apparatus according to the prior art, has a passage section considerably larger, which allows to overcome all the ventilation problems previously present.

Moreover, thanks to the methods described, for the performance of the method will not be necessary to use a curved needle, but a simple cannula-needle, preferably of 14G.

Further important point to note is the fact that, through the introduction of the oro-tracheal tube 10 according to the present invention, not only the steps of the method of Fantoni are simplified, but also, unique and important, it is possible to have a direct vision of all steps.

In summary, the oro-tracheal tube according to the present invention, in the embodiment which comprises at least two lumens, makes the interventions of tracheotomy, according to any known method (dilated or percutaneous Ciaglia, translaryngeal or Fantoni, surgical), executable easily even in the hands of inexperienced doctors, not requiring any time issue, allowing to follow the entire procedure under direct vision, until the proper placement of the cannula, working in a surgical field always very clean, free from secretions and blood aspirated from the suction system:

In the following the benefits will be discussed specifically arising from the use of the oro-tracheal tube according to the present invention in the execution of tracheotomies performed according to the different techniques of the prior art. All these techniques of tracheotomy are valid, but all have critical moments that can compromise the patient's life or the failure of the tracheotomy itself.

With regard to the technique of percutaneous dilated tracheostomy (Ciaglia), the insertion of a fiber optic system (bronchoscope or similar systems such as a videomandrel) takes place directly through the orotracheal tube with which, according to the prior art, the patient is ventilated, greatly reducing the ventilatory surface and impairing the seal of the tube, for which the mixture of air + O₂ insufflated into the lungs, already with difficultly, in part protrudes from the port of entry of the fiber optic system, leading to a considerable decrease in ventilatory ability.

This all results in poor ventilation of the patient, becoming almost always necessary the assistance in manual ventilation (requiring the presence of a dedicated operator).

In contrast, the oro-tracheal tube according to the present invention, being provided with an exclusively ventilatory lumen, is connected directly to the mechanical ventilator and to its inside is not entered any type of equipment. The asymmetric headset present in its distal end, inflated, above the hull, allows to ventilate the patient's lungs in safety and with no leakage. The oro-tracheal tube according to the invention can thus be drawn as any oro-tracheal tube according to the prior art and can always be connected to the respirator, assisting the patient always in mechanical ventilation, thereby eliminating the aid of an operator, ensuring however, optimal ventilation.

A second problem of the technique of percutaneous dilated tracheostomy (Ciaglia) implemented using devices according to the prior art resides in the fact that the output of the fiber optic system from the oro tracheal tube, necessary to obtain the transillumination in the area where the stoma is to be practiced, that is, between the first and second or between the second and third tracheal ring, is normally much lower, usually in the distal third of the tracheal. Consequently, to exit from the tube to obtain the transillumination in the right area of the trachea, it is necessary to displace the oro-tracheal tube according to the prior art with the headset that rises above the vocal cords, a position which does not ensure a good ventilation, and moreover which implies that the tube is no longer anchored below the vocal cords and is complicated to try to keep it with the final part between the vocal cords themselves, losing the perfect adhesion obtained with the cuff inflated below the vocal cords, with difficulties in ventilate the patient as a large part of the air blown in to the lungs does not come back out of the mouth; also it is necessary the manual intervention of an operator dedicated to hold the tube in some way.

Moreover, even in this case it is impossible to ventilate on mechanical ventilation (VAM), but it is necessary to ventilate the patient with manual circuit.

Moreover, to displace the tube in this completely ineffective position is necessary, as well as for the transillumination, also to avoid that when piercing the trachea with the cannula also the tube can be perforated, the contemporary perforation of the trachea and the tube below representing, in effect, one of the most frequent complications arising from the application of this method with an oro-tracheal tube according to the prior art.

By contrast, the use of the oro-tracheal tube according to the present invention avoids all these drawbacks. In fact, the oro-tracheal tube according to the invention can be positioned in such a way that the output of the ventilatory lumen (ie the distal end of the rear tubular element 10') is in proximity of the hull, while the operative lumen (ie the distal end of the front tubular element 10") ends at the rider between the vocal cords. The fiber optic system, inserted through the operative lumen, slides over the vocal cords into the trachea, just between the first and second tracheal ring, where in fact it is necessary to obtain the transillumination and where to practice the stoma.

The insertion of the cannula-needle into the trachea at the level of the area subject to transillumination, occurs in an area of the trachea free from the front tubular element 10", therefore it is impossible to pierce the tube.

After it is positioned correctly, and without having to move it, the oro-tracheal tube according to the present invention, therefore, allows a correct and continuous mechanical ventilation of the patient. It also protects the rear wall of the trachea from the possible trauma that often occurs during the procedures to penetrate into the trachea and to perform the expansion and at the same time allows, through the operative lumen, to easily insert the lens into the trachea (at the height of the first-second tracheal ring) and perform all the maneuvers necessary to perform the stoma under direct vision, with the final insertion of the cannula, in perfect safety, without limits of time, not interfering at all with the ventilation of the patient.

Furthermore, due to the inflation of the cuff disposed in the vicinity of the distal end of the rear tubular element 10', the oro-tracheal tube according to the present invention allows the full protection of the lower airways against both possible bleeding and secretions.

The particular characteristics of the oro-tracheal tube according to the present invention allow to introduce some modifications to other elements of the kit of translaryngeal tracheostomy.

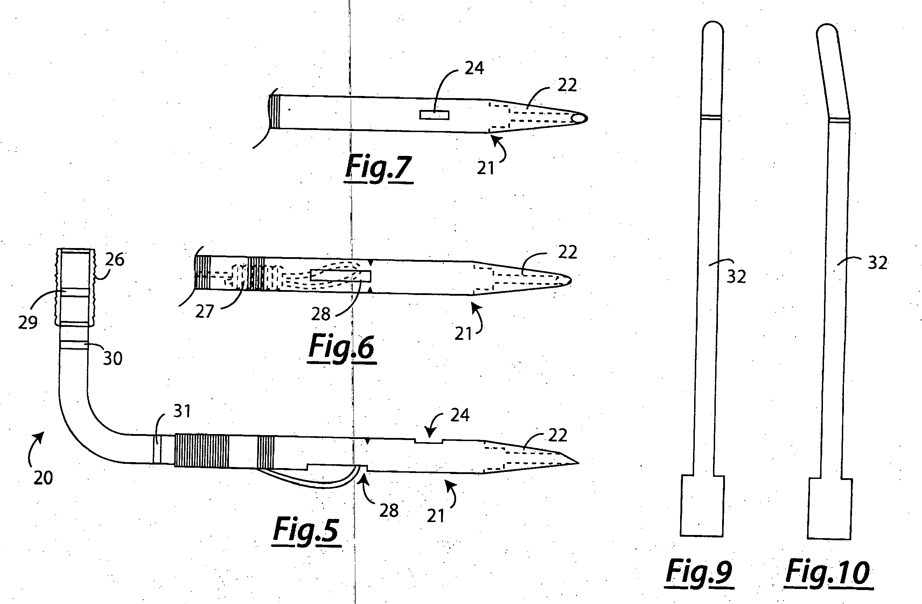

In particular, to complete the modification, also to reduce the production costs of the equipment as a whole, the same tracheal cannula of Fantoni was modified, as shown with reference to Figures 5, 6 and 7, in which the tracheal cannula is designated as a whole by the reference numeral 20 and the removable element of introduction by the reference number 21.

Differently from the equipment of the prior art, the tip 22 of the removable element of introduction 21 is not made of steel, to assemble with the rest, but is represented by the same terminal part of the cone of the removable element of introduction 21, made of the same plastic material suitably shaped to ensure greater hardness and rigidity. The tip 22 terminates slant, and is provided with a central channel 23, preferably of a diameter of 14G (1.628 mm) for the passage of the guidewire.

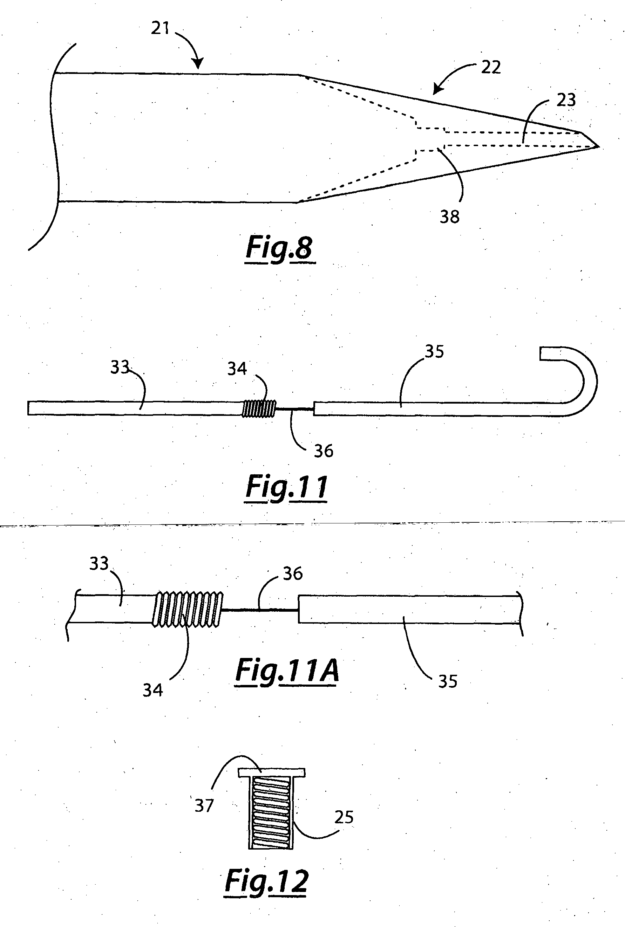

On the upper wall, the introducing member 21 is removably provided with an opening 24, preferably of 1 cm in length, which serves for the recovery of the guide wire 33, after which the same is introduced through the cannula-needle in the trachea and brought out of the oral cavity through the lumen defined by the front tubular element 10"of the oro-tracheal tube 10 and being inserted into the channel 23 of the tip 22 of the cannula 20. At this point, and unlike the prior art, the wire guide 33 does not have to exit at the opposite end of the cannula 20, but, through said opening, can safely be recovered and separated from the guide 35 by the Seldinger wire guide 33, as will be better illustrated with reference to Figures 11, 11A and 12, and then a fixing screw 25 is screwed which serves as a wholesale for traction.

Inside, the tip 22 of the removable element of introduction 21 is shaped so as to provide a seat for the enlarged head 37 of the fixing screw 25 (Figure 12) of the guide wire 33.

No longer having the guide wire exit from the opposite end of the cannula 20, it is possible to provide the cannula 20 of a headset 26, placed at the opposite end of the cannula 20 with respect to the tip 22 of the introduction element 21, and provided with a system of full inflation of tank 27 with one-way valve for the air, already connected, housed entirely within the cannula itself. Once out of the introducing member 21 and part of the cannula 20 from the trachea to the outside of the neck, it will be sufficient, through said opening 24, to extract the inflation system from the cannula 20, through an opening 28 which is located in the lower element 21 of the cannula 20 and measuring about 2cm in length. Once extracted, the inflation system is fully functional and ready for use. This has obvious advantages compared to the solutions of the prior art, in which the inflation system could not be supplied already inserted in the cannula 20, since the inner lumen of the cannula 20 must remain free to allow the passage of the thread 33, inserted from the tip 22 of the cannula, to recover it from the opposite end of the cannula to perform the node necessary to create the previously said enlargement.

This possibility is at the same time a simplification of the construction and results in a reduction of costs.

In addition, the tracheal cannula 20 is provided with colored marks used as reference points, respectively a first notch 29 of indication of the first level with respect to the skin, ie the level at which the removable introducing member 21 of the cannula 20 is cut and and the valving element 32 required to make rectilinear the cannula during its revolution within the trachea is inserted into the cannula itself; a second notch 30 (visible on the skin) of indication of the second level with respect to the skin, which marks the limit of the final traction and a third mark 31 (visible from the trachea through the bronchoscope) to indicate the starting level for the revolution and the final slot of the cannula.

Figures 9 and 10 show respectively a top view and a side view of a shutter 32 of an apparatus of translaryngeal tracheostomy, which presents in the final stretch an angle of 30 degrees, unlike the shutters according to the prior art, which are completely straight.

The use of the shutter 32 is made unnecessary by the fact that the cannula 20 is made of a material with shape memory, which forms the cannula 20 to assume its conformation angled only persisting at a temperature equal to body temperature, ie at least 37° C, which occurs after the application of cannula 20 into the trachea. The realization of the cannula 20 with this material would reduce its size throughout the operating act, allowing to best use the bronchoscopic tube 10' according to the embodiment of the present invention shown with reference to Figure 2, ie in the variant provided with a double cuff. In addition, the cannula made with this material can be of small size, its turning and positioning would be facilitated.

The guide wire 33 has also been modified (Figures 11, 11A, 12), developing a system of male-female screwing, which avoids the use of a node used in accordance with the prior art, which is cumbersome and difficult to perform.

In particular, the guide wire 33 is provided at its one end 34 of a Seldinger type guide 35 witch a J-tip, coupled to the guide wire of steel by means of a thin wire coupling 36, made of copper, and the surface of said end 34 of the (guide) wire 33 is threaded for coupling with said means of enlarged.

After the introduction of the Seldinger type guide in the removable element of introduction 21 of the tracheal cannula 20, the coupling wire 36 is cut and the Seldinger type guide 35 is removed. Then, a fixing screw 25 is screwed at the end 34 of the threaded steel guidewire 33. The fixing screw 25 has an enlarged head 37, which engages in the corresponding seat 38 inside of the removable element of introduction 21 of the tracheal cannula 20, so as to become jammed within the cannula 20, allowing the traction.

In conclusion, the new technique is an advantage over the prior art, allowing to obtain a particular ease of execution, a high safety for the patient (direct visual control of all phases of the maneuver), a reduction of costs.

The present invention has been described for illustrative but not limitative purposes, according to its preferred embodiments, but it is to be understood that variations and/or modifications can be made by those skilled in the art without departing from the scope of protection as defined by the appended claims.
1. Colombo E. Le tracheotomie. AOOI, quaderni di aggiornamento
2. Grillo H.Development of TrachealSurgery: A HistoricalReview. Part 1: Techniques of Tracheal Surgery. AnnThoracSurg 2003;75:610-9
3. Rana S, Pendem S. Tracheotomy in critical ill patients. Mayo ClinProc 2005; 80 (12) 1632-38.
4. Petros S. Percutaneous tracheostomy. Crit Care 1999, 3 R5-R10
5. Meininger D Byhahn C. Translaryngeal tracheotomy. Operative Techniques in Otolaryngology 2007, 18: 99-104.
6. De Lyen P. Bedert L. Tracheotomy: clinical review and guidelines. Europ J Cardio-thoracicSurg 2007, 32: 417-421.
7. Consensus Conference on Artificial Airways in patient receiving mechanichal ventilation. Chest 1989, 96, 1
8. King C, Moores L. Controversies in mechanical ventilation: when should a tracheotomy be placed? ClinChestMed 2008, 29: 253-63.
9. Davis K, Campbell RS. Changes in respiratory mechanics after tracheostomy. ArchSurg 1999; 134: 59-62.
10. Diehl JL, Lemaire F, Brochard L. Changes in the work of breathing induced by tracheotomy in ventilator-dipendent patient. Am J RespCrit Care Med 1999; 159:383-8.
11. Freeman BD, Isabella K. A prospective, randomized study comparing percutaneous with surgical tracheostomy in critically ill patients. Crit Care Med 2001; 29,5
12. Freeman BD, Isabella K. A meta-analysis of prospective trials comparing percutaneous and surgical tracheostomy in critically ill patients. Chest 2000; 1118:1421
13. Cooper RM. Use and safety of percutaneous tracheostomy in intensive care. Anasthesiology 1998; 53:1209-27
14. Ciaglia P, Firsching R. Elective percutaneous dilatational tracheostomy. A new simple bedside procedure; preliminary report. Chest 1985; 87:715-19.
15. Trottier SJ, Hazard PB. Posterior tracheal wall perforating during percutaneous dilational tracheostomy. Chest 1999; 115,
16. Kost K. Endoscopic percutaneous dilatational tracheotomy: a prospective evaluation of 500 consecutive cases. Laryngoscope 2005; 115 (oct) 1-30.
17. Fikkers G. Percutaneous tracheostomy with the Blue-rhino tecnique. Anaesthesia, 2002; 57:1094-97.
18. Fikkers G. Comparison of two percutaneous tracheostomy techniques, guide wire dilating forceps and Ciaglia Blue-Rhino: a sequential cohort study. Crit Care Med 2004; vol8 n°5.
19. Griggs WM, Worteley LIG. A simple percutaneous tracheostomy technique. SurgGynecolObstet 1990; 170: 543-5.
20. Park SS, Oroenberg D. Percutaneous tracheotomy: griggs technique. Operat Technique in Otol 2007; 18: 95-98.
21. ParanH. Evaluation of modified percutaneous tracheostomy technique without bronchoscopic guidance. Chest 2004; 126:868-81.
22. Fantoni A. Ripamonti D. A non-derivative, non-surgical tracheostomy: the translaryngeal method. Intensive Care Med 1997, 23: 386-92.
23. Nani R, Sarpellon M. tracheotomia translaringea secondo Fantoni. Minerva Anest 2002, vol.68 n°3.

## Claims

1. Oro-tracheal tube (10) for interventions of tracheotomy wherein it is divided into two separated lumens, each being open at both its ends, a first lumen that reaches the end of the distal portion (12) of the tube (10) and is situated to the rear along the sagittal axis with respect a second lumen that reaches an intermediate portion of the overall length of staid oro-tracheal tube (10), **characterized in that** at least in the distal portion (12) it has a flattened section, more extended along the transverse axis and less extended along the sagittal axis, and that, close to the end of said distal portion it is provided with asymmetric expandable means, which expand mainly towards the front.

2. Oro-tracheal tube (10) according to claim 1, **characterized in that** said asymmetric expandable means are selected from: an inflatable cuff (13), a ring of self-expandable or shape memory material.

3. Oro-tracheal tube (10) according to claim 1 or 2, **characterized by** the fact of being preformed with a physiological curvature in the longitudinal direction.

4. Oro-tracheal tube (10) according to any one of the preceding claims, **characterized in that**, at least in the distal portion (12), it has a crescent-shaped section, with the front side being concave and the back side being convex.

5. Oro-tracheal tube (10) according to any one of the preceding claims, **characterized in that** said second lumen is in turn divided into two lumens.

6. Kit for tracheotomy comprising a oro-tracheal tube as defined in claims 1-5.

## Patentansprüche

1. Orotrachealer Tubus (10) für Tracheotomie-Eingriffe, wobei er in zwei getrennte Lumina unterteilt ist, die jeweils an beiden Enden offen sind, ein erstes Lumen, das das Ende des distalen Abschnitts (12) des Schlauchs (10) erreicht und sich auf der hinteren Seite entlang der sagittalen Achse mit Bezug auf ein zweites Lumen befindet, das einen intermediären Abschnitt der Gesamtlänge des orotrachealen Schlauchs (10) erreicht, **dadurch gekennzeichnet, dass** er zumindest im distalen Teil (12) einen abgeflachten Abschnitt hat, stärker ausgedehnt entlang der transversalen Achse und weniger ausgedehnt entlang der sagittalen Achse, und dadurch, dass er nahe dem Ende des distalen Teils mit asymmetrischen expandierbaren Mitteln ausgestattet ist, die hauptsächlich nach vorne expandieren.

2. Orotrachealer Tubus (10) gemäß Anspruch 1, **dadurch gekennzeichnet, dass** die asymmetrischen expandierbaren Mittel gewählt sind aus: einer aufblasbaren Manschette (13), einem Ring aus selbst-expandierbarem oder Formgedächtnismaterial.

3. Orotrachealer Tubus (10) gemäß Anspruch 1 oder 2, **dadurch gekennzeichnet, dass** er mit einer physiologischen Krümmung in der Längsrichtung vorgeformt ist.

4. Orotrachealer Tubus (10) gemäß einem beliebigen der obigen Ansprüche, **dadurch gekennzeichnet, dass** er, zumindest im distalen Teil (12), einen sichelförmigen Abschnitt hat, dessen Vorderseite konkav und dessen Rückseite konvex ist.

5. Orotrachealer Tubus (10) gemäß einem beliebigen der obigen Ansprüche, **dadurch gekennzeichnet, dass** das zweite Lumen wiederum in zwei Lumina unterteilt ist.

6. Kit zur Tracheotomie, das einen orotrachealen Tubus wie in Anspruch 1-5 definiert umfasst.

## Revendications

1. Tube orotrachéal (10) pour des interventions de trachéotomie, lequel est divisé en deux lumières séparées, chacune étant ouverte à ses deux extrémités, une première lumière qui atteint l'extrémité de la partie distale (12) du tube (10) et se trouve à l'arrière sur l'axe sagittal par rapport à une seconde lumière qui atteint une partie intermédiaire de la longueur globale dudit tube orotrachéal (10), **caractérisé en ce que**, au moins dans la partie distale (12), il a une portion aplatie qui s'étend davantage sur l'axe transversal et s'étend moins sur l'axe sagittal, et **en ce que**, près de l'extrémité de ladite partie distale, il comporte des moyens extensibles asymétriques qui s'étendent principalement vers l'avant.

2. Tube orotrachéal (10) selon la revendication 1, **caractérisé en ce que** lesdits moyens extensibles asymétriques sont choisis parmi : une manchette gonflable (13), un anneau de matière auto-extensible ou à mémoire de forme.

3. Tube orotrachéal (10) selon la revendication 1 ou 2, **caractérisé en ce qu'**il est préformé avec une courbure physiologique dans le sens longitudinal.

4. Tube orotrachéal (10) selon l'une quelconque des revendications précédentes, **caractérisé en ce que**, au moins dans la partie distale (12), il a une section en forme de croissant, le côté avant étant concave et le côté arrière étant convexe.

5. Tube orotrachéal (10) selon l'une quelconque des revendications précédentes, **caractérisé en ce que** ladite seconde lumière est elle-même divisée en deux lumières.

6. Kit pour trachéotomie comportant un tube orotrachéal selon les revendications 1 à 5.
